# EUROPEAN PATENT SPECIFICATION

(11) **EP 1 981 982 B1**
(45) Date of publication and mention of the grant of the patent: **27.04.2011**
(21) Application number: 07714324.6
(22) Date of filing: 09.02.2007
(51) Int. Cl.: C12P 7/64, C12P 9/00, C12P 13/00

(54) **MICROBIAL FERMENTATION-BASED PRODUCTION OF PHOSPHOLIPIDS CONTAINING LONG-CHAIN POLYUNSATURATED FATTY ACIDS AS THEIR CONSTITUENTS**
AUF MIKROBIELLER FERMENTATION BERUHENDE PRODUKTION VON PHOSPHOLIPIDEN MIT LANGKETTIGEN MEHRFACH UNGESÄTTIGTEN FETTSÄUREN ALS BESTANDTEILEN
PROCÉDE BASÉ SUR LA FERMENTATION MICROBIENNE DE PRODUCTION DE PHOSPHOLIPIDES CONTENANT EN TANT QUE CONSTITUANTS DES ACIDES GRAS POLYINSATURÉS À LONGUE CHAÎNE

(30) Priority: 10.02.2006 JP 2006033657
(43) Date of publication of application: 22.10.2008
(73) Proprietor: Suntory Holdings Limited, Kita-ku, Osaka-shi Osaka 530-8203 (JP)
(72) Inventor: SHIRAISHI, Akiko, Ibaraki-shi, Osaka 567-0829 (JP); KAWASHIMA, Hiroshi, Takatsuki-shi, Osaka 569-1121 (JP)
(74) Representative: Vossius & Partner
(86) International application number: PCT/JP2007/052794
(87) International publication number: WO 2007/091731

(56) References cited:
- EP-A- 1 498 488
- EP-A- 1 731 616
- EP-A1- 0 960 943
- LINDBERG A ET AL: "EFFECT OF TEMPERATURE AND GLUCOSE SUPPLY ON THE PRODUCTION OF POLYUNSATURATED FATTY ACIDS BY THE FUNGUS MORTIERELLA ALPINA CBC 343.66 IN FERMENTOR CULTURES" APPLIED MICROBIOLOGY AND BIOTECHNOLOGY, SPRINGER VERLAG, BERLIN, DE, vol. 39, 1993, pages 450-455, XP002924350 ISSN: 0175-7598
- HIGASHIYAMA K ET AL: "PRODUCTION OF ARACHIDONIC ACID BY MORTIERELLA FUNGI" BIOTECHNOLOGY AND BIOPROCESS ENGINEERING, KOREAN SOCIETY FOR BIOTECHNOLOGY AND BIOENGINEERING, SEOUL, KR, vol. 7, no. 5, September 2002 (2002-09), pages 252-262, XP009025999 ISSN: 1226-8372
- JAREONKITMONGKOL SAEREE ET AL: "A novel DELTA-5-desaturase-defective mutant of Mortierella alpina 1S-4 and its dihomo-gamma-linolenic acid productivity" APPLIED AND ENVIRONMENTAL MICROBIOLOGY, vol. 59, no. 12, 1993, pages 4300-4304, XP002437027 ISSN: 0099-2240

## Description

The present invention relates to a method for producing a phospholipid containing a C₂₀ or higher long-chain polyunsaturated fatty acid as a constituent (i.e., long-chain polyunsaturated fatty acid-phospholipid; LCPUFA-PL), wherein said method comprises culturing a microorganism belonging to *Mortierella sp.* under conditions allowing increased intracellular accumulation of LCPUFA-PL in the microorganism, and extracting LCPUFA-PL from the cultured product of the microorganism.

Phospholipids (PL) are known to have various physiological functions, such as an improving effect on brain functions, an anti-stress effect, and a cholesterollowering effect. There are several types of PLs, including, mainly, phosphatidylcholine (PC), phosphatidylserine (PS), phosphatidylethanolamine (PE) and phosphatidylinositol (PI). These PLs have mutually different functions and physical properties.

Among PLs, those containing a C₂₀ or higher long-chain polyunsaturated fatty acid (abbreviated as "LCPUFA") as a constituent (for convenience of explanation, such phospholipids are abbreviated as "LCPUFA-PL") have a high improving effect on brain functions, and hence have higher effects than phospholipids containing no LCPUFA as a constituent (for convenience of explanation, abbreviated as "non-LCPUFA-PL") (see Non-patent Document 1). Specific examples of LCPUFA include, for example, docosahexaenoic acid (DHA) and arachidonic acid (ARA).

Likewise, LCPUFA derivatives of non-phospholipid type are also known to have an improving effect on brain functions (see Patent Document 1). However, in LCPUFA derivatives of non-phospholipid type, their improving effect on brain functions appears to be mediated by their action on the hippocampus in the cerebrum, unlike the above phospholipids including LCPUFA-PL and non-LCPUFA-PL.

Reasons that LCPUFA-PL has a higher improving effect on brain functions include: (1) LCPUFA-PL is a structure actually present in the brain; (2) LCPUFA-PL can pass through the blood-brain barrier; and (3) LCPUFA-PL reaches a target tissue (e.g., brain) without being captured or modified in the liver because its absorption is not mediated by the liver.

Detailed explanation of each reason will be given below. First, as for Reason (1), LCPUFA in the brain is known to exist predominantly in phospholipid form. More specifically, LCPUFA exists mainly as a compound such as PC, PS, PE or PI shown above and exhibits various functions in the brain. Secondly, as for Reason (2), phospholipids have been found to reach brain tissues because labeled phospholipids when taken orally are detectable in brain tissues (see Non-patent Document 2). Further, as for Reason (3), during phospholipid absorption, one of the two constituting fatty acids is hydrolyzed in the digestive organs to produce a lysophospholipid. This lysophospholipid is absorbed by the small intestine and reconstructed into a phospholipid in small intestine cells, which is then absorbed through lymph vessels (see Non-patent Document 3). For this reason, LCPUFA-PL is delivered throughout the body without passing through the liver.

LCPUFA-PL has been conventionally produced through preparation or purification from materials rich in LCPUFA-PL, such as animal organs and eggs. Specific examples include preparation from bovine brains and purification of phospholipid fractions from pig livers or fish eggs (see Patent Documents 2 and 3). It has also been known that certain kinds of marine bacteria produce LCPUFA-PL (see Non-patent Document 4), and that filamentous fungi having the ability to produce arachidonic acid contain arachidonic acid-containing phospholipids in their cells (see Non-patent Document 5).

In current techniques for industrial production of phospholipids, phospholipids are usually extracted together with fats rich in triglycerides when extracted from source materials. Solvents used for extraction include hexane. The extracted fats contain a gummy matter, which causes discoloration and foaming of the fats. Thus, such a gummy matter should be removed in a degumming step; but almost all phospholipids are transferred to the gummy matter during this step. Phospholipids are therefore produced by purifying the gummy matter.

However, LCPUFA-PL provided by the above conventional techniques neither achieves stable supply nor always ensures reliable quality. More specifically, LCPUFA-PL currently available is generally derived from materials as mentioned above, including animal organs, egg yolk and fish eggs. These animal and vegetable oil-derived sources are associated with problems such as weather-induced variations in their supply and LCPUFA content, as well as influences caused by environmental pollution. Moreover, there are great difficulties associated with the use of animal organs including bovine brains, following the epidemic of bovine spongiform encephalopathy. For these reasons, the conventional techniques are subject to a problem in that it is difficult to ensure efficient and stable production of LCPUFA-PL.

In the case of using microbial techniques, LCPUFA-PL derived from marine bacteria is not suitable as a nutrient composition because it contains branched fatty acids as its major components, which are hardly found in humans and other animals, and are unique to bacteria.

On the other hand, there is proposed LCPUFA-PL production using *Conidiobolus sp.,* a member of filamentous fungi that produce arachidonic acid (see Patent Document 4), but this strain is reported to contain large amounts of branches fatty acids such as isomyristic acid (iso14:0) and isopalmitic acid (iso16:0) (see Non-patent Documents 6 and 7). Moreover, some fungi may cause a mycosis unique to *entomophthorales* (called entopmophthorosis, a kind of mycosis, which is first developed in intranasal mucous membranes and then expanded over the pharynx, intranasal airway and whole face), and other fungi have the ability to cause infections in healthy people (Biosafety Level 2) (see Non-patent Documents 8 and 9). Furthermore, such fungi are likely to be parasitic on insects and other small animals (see Non-patent Document 10) and hence are not suitable as industrially used microorganisms in terms of their adverse effects on humans and the environment.

Likewise, in *Mortierella sp*. which are currently used for industrial production of arachidonic acid-containing triglycerides, active efforts have been made to examine various conditions for arachidonic acid production. Above all, it has been reported that addition of various mineral salts and/or conditions including medium pH and culture temperature improve the productivity of arachidonic acid. More specifically, addition of various mineral salts has been found to have an effect on the productivity of both LCPUFA-containing triglycerides and LCPUFA-PL accumulated in microbial cells (Non-patent Document 11). With respect to the initial pH, it is known that (1) the initial pH optimum for culturing is 6.0 to 6.6 (Non-patent Document 11), and that (2) the yield of arachidonic acid-containing triglycerides is increased when elevating the pH in the lathe stage of culturing (Patent Document 5). However, there is no knowledge optimized for phospholipid fractions. Further, with respect to the culture temperature, it is known that the LCPUFA content in phospholipids is increased by culturing at low temperature for 5 or more days (Non-patent Document 12). However, such long-term culturing at low temperature is not suitable for industrial production in view of cost, and all of the conditions stated above focus on the production of LCPUFA present as a constituent of triglycerides. Thus, culture conditions specific for LCPUFA-PL production have not yet been studied under the present circumstances. In microbial cells produced by these culturing techniques, LCPUFA-PL is detected as a minor component mixed into considerable amounts of triglycerides, and its content is very low (11.7 mg/g of dried cells; see Non-patent Document 5). Moreover, since LCPUFA-PL is a byproduct of triglyceride production, its quality and supply are not stabilized. Thus, there is no expectation for sufficient use of LCPUFA-PL.

In view of the foregoing, there has been a demand for the development of LCPUFA-PL-containing fats that can be used more safely and more stably for foods and animal feed.
[Patent Document 1] JP 2003-48831 A (published on February 21, 2003)
[Patent Document 2] JP 11-35587 A (published on February 9, 1999)
[Patent Document 3] JP 8-59678 A (published on March 5, 1996)
[Patent Document 4] JP 6-2068 B (published on January 12, 1994)
[Patent Document 5] Japanese Patent Domestic Announcement No. 10-512444 (published on December 2, 1998)
[Non-patent Document 1] A. Bruni, et al., Nature, Vol. 260, p331-333 (1976)
[Non-patent Document 2] G. Toffano, et al. Clinical Trials Journal, Vol. 24, p18-24 (1987)
[Non-patent Document 3] Katsumi Imaizumi, Rinsyo-Eiyo (Clinical Nutrition), Vol. 67, p119 (1985)
[Non-patent Document 4] Kazuyoshi Yazawa, et al., Yukagaku (Oil Science), Vol. 44, p787-793 (1995)
[Non-patent Document 5] S. Shimizu, et al., JAOCS, Vol. 68, No. 4, p254-258 (1991)
[Non-patent Document 6] D. Tyrrell, Can. J. Microbiol., Vol. 17, p1115-1118 (1971)
[Non-patent Document 7] D. Tyrrell, et al., Can. J. Microbiol., Vol. 22, p1058-1060 (1976)
[Non-patent Document 8] G. S. de Hoog, et al., ATLAS OF CLINICAL FUNGI, 2nd edition, p.118-123
[Non-patent Document 9] Japanese Society for Bacteriology, Biosafety Guideline for Pathogenic Bacteria, revised 2nd edition, p7 (2002)
[Non-patent Document 10] Takeharu Hasegawa, et al., Classification and Identification of Microorganisms, p19-20, University of Tokyo Press (1975)
[Non-patent Document 11] K. Higashiyama, et al., JAOCS, Vol. 75, p1501-1505 (1998)
[Non-patent Document 12] S. Jareonkitmongkol, et al., Arch. Microbiol., Vol. 161, p316-319 (1994)

EP 1 731 616 A1 discloses a process for producing phospholipid containing long chain polyunsaturated fatty acid as constituent thereof and utilisation of the same.

EP 0 960 943 A1 relates to media for culturing microorganisms and a process for producing unsaturated fatty acids or lipids containing the same.

EP 1 498 488 A1 discloses a process for producing highly unsaturated fatty acid-containing lipid.

The object of the present invention is to provide a technique that enables efficient and stable supply of LCPUFA-PL of a reliable quality for safe use in foods and animal feed. This technique is achieved by obtaining a cultured product of a microorganism which accumulates a high content of a phospholipid containing a long-chain polyunsaturated fatty acid as a constituent (LCPUFA-PL), whose content of branched fatty acids is low.

To overcome the problems stated above, the inventors of the present invention have made efforts to develop a method for efficient LCPUFA-PL production with a low content of branched fatty acids. As a result, the inventors have found that *Mortierella sp*. are preferred for this purpose as lipid-producing microorganisms which produce long-chain polyunsaturated fatty acid (LCPUFA)-containing lipids.

Moreover, in spite of predicting that LCPUFA-PL is strictly controlled for its intracellular synthesis due to it being a constituent of biomembranes, the inventors have found conditions under which the amount of LCPUFA-PL per cell can be significantly increased, and have completed the present invention.

Namely, the present invention provides a method for producing a phospholipid containing LCPUFA as a constituent (LCPUFA-PL), whose content of branched fatty acids is low, wherein the above lipid-producing microorganisms are cultured to ensure increased accumulation of LCPUFA-PL in their cells. The above method further comprises, after the step of culturing the above lipid-producing microorganisms, a step of obtaining dried cells available for use as a supply source of LCPUFA-PL, and a fat extraction step where LCPUFA-PL-containing fats are extracted from the cells.

Thus, the method of the present invention is a method for producing LCPUFA-PL, which comprises:
(i) culturing a microorganism belonging to *Mortierella sp*. wherein the culture is controlled to maintain between ph 4 and 6, thereby allowing intracellular accumulation of LCPUFA-PL in the microorganism; and
(ii) obtaining LCPUFA-PL from the cultured product of the microorganism, wherein the compositional ratio of isopalmitic acid, a branched fatty acid, is 5% or less based on the total amount of fatty acids contained as constituents of all phospholipids (PL) contained in the cultured product.

Alternatively, the method of the present invention is a method for producing LCPUFA-PL, which comprises:
(i) culturing a microorganism belonging to *Mortierella sp.* wherein the culture is controlled to maintain between ph 4 and 6, thereby allowing intracellular accumulation of LCPUFA-PL in the microorganism;
(ii) extracting lipid components as a total lipid fraction from the cultured product of the microorganism, followed by fractionation into triglyceride and phospholipid fractions; and
(iii) obtaining LCPUFA-PL from the phospholipid fraction, wherein the compositional ratio of isopalmitic acid, a branched fatty acid, is 5% or less based on the total amount of fatty acids contained as constituents of all phospholipids (PL) contained in the cultured product.

Microorganisms used in the method of the present invention are not limited in any way as long as they are microorganisms belonging to *Mortierella sp.* and are capable of producing LCPUFA-PL. Microorganisms of *Mortierella sp.* used in the method of the present invention are preferably those of the *Mortierella* subgenera. Microorganisms of the *Mortierella* subgenera include *Mortieralla alpina, Mortierella polycephala, Mortierella exigua, Mortierella hygrophila,* and *Mortierella elongata.* A more preferred microorganism of *Mortierella sp*.- used in the method of the present invention is *Mortierella alpina.*

As to microorganisms belonging to *Mortierella sp.,* there are many species and strains known to have the ability to produce LCPUFA-PL containing arachidonic acid (ARA) as its constituent LCPUFA (abbreviated as "ARA-PL"). When selected from *Mortierella sp.* having the ability to produce ARA-PL, the microorganisms of *Mortierella sp.* are preferably those of the *Mortierella* subgenera. Microorganisms of the *Mortierella* subgenera include *Mortierella alpina, Mortierella polycephala, Mortierella exigua, Mortieralla hygrophila,* and *Mortierella elongata.*

More specific strains of microorganisms of the *Mortierella* subgenera having the ability to produce ARA-PL include, for example, *Mortierella polycephala* (*M. polycephala*) IFO6335, *Mortierella elongata* (*M. elongata*) CBS125.71, *Mortierella exigua* (*M*. *exigua*) IFO8571, *Mortierella beljakovae* (*M. beljakovae*) CBS601.68, *Mortierella schmuckeri* (*M. schmuckeri*) NRRL2761, *Mortierella hygrophila* (*M. hygrophila*) IFO5941, as well as *mortierella alpina* (*M. alpina*) IFO8568, ATCC16266, ATCC32221, ATCC42430, CBS219.35, CBS224.37, CBS250.53, CBS343.66, CBS527.72, CBS529.72, CHS608.70 and CBS754.68. All of these strains are available without any restriction from the Institute for Fermentation, Osaka (IFO), American Type Culture Collection (ATCC) and Centrralbureau voor Schimmelcultures (CBS). Further, other strains of the *Mortierellia* subgenera having the ability to produce ARA-PL include *Mortierella elongata* (*M. elongata*) SAM0219 (FERM P-8703, FERM BP-1239), a strain isolated from soil by the research group including the inventors of the present invention.

Likewise, more specific strains of microorganisms having the ability to produce LCPUFA-PL containing dihomo-γ-linolenic acid (DGLA) as its constituent LCPUFA - (abbreviated as "DGLA-PL") include *Mortierella elongata* (*M. elongata*) SAM1860 (FERM BP-3589), a strain isolated from soil by the research group including the inventors of the present invention.

Further, more specific strains of microorganisms having the ability to produce LCPUFA-PL containing Mead acid as its constituent LCPUFA (abbreviated as "Mead acid-PL") include *Mortierella alpina* (*M. alpina*) SAM2086 (FERM P-15766), a strain isolated from soil by the research group including the inventors of the present invention.

These strains of the microorganisms belonging to *Mortierella sp.* used in the method of the present invention may be selected for use alone or in combination, depending on the type of LCPUFA to be produced.

In this specification, the microorganisms used in the method of the present invention may also be referred to as "lipid-producing microorganisms" and simply as "fungi". The terms are synonymous herein.

Culture conditions for the microorganisms used in the method of the present invention are not limited in any way and may be determined, as appropriate, depending on the type of strain to be cultured, as long as the culture is controlled to maintain between ph 4 and 6.

To culture microorganisms including the above strains used in the present invention, spores, hyphae or a precultured solution of each strain is/are inoculated into a liquid medium or onto a solid medium and then cultured. In the case of a liquid medium, any commonly used carbon source may be used, including, but not limited to, glucose, fructose, xylose, saccharose, maltose, soluble starch, molasses, glycerol and mannitol. As a nitrogen source, it is possible to use a natural nitrogen source such as peptone, yeast extract, malt extract, meat extract, casamino acid, corn steep liquor, soybean protein, defatted soybean meal or cottonseed meal, as well as an organic nitrogen source such as urea and an inorganic nitrogen source such as sodium nitrate, ammonium nitrate or ammonium sulfate. Particularly preferred are soybean-derived nitrogen sources, as specifically exemplified by soybeans, defatted soybean meal, soybean flakes, edible soybean protein, soybean fiber, soy milk, soybean powder, etc. Likewise, defatted soybean meal which is thermally denatured, and more preferably that which is thermally treated at about 70°C to 90°C and further treated to remove ethanol-soluble components, may be used either alone or in combination, or in combination with any nitrogen source listed above. For practical purposes, in general, the total amount of carbon sources to be added is preferably within a range between 0.1% and 40% by weight, more preferably within a range between 1% and 25% by weight. Likewise, the total amount of nitrogen sources to be added is preferably within a range between 0.01% and 10% by weight, more preferably within a range between 0.1% and 10% by weight. Further, in the case of a fed-batch medium, it is preferred that the initial amount of carbon sources to be added is set within a range between 1% and 5% by weight, while the initial amount of nitrogen sources to be added is set within a range between 0.1% and 6% by weight. While medium ingredients to be added during culturing may consist of both carbon and nitrogen sources, it is more preferable to add only a carbon source.

Ingredients other than the above carbon and nitrogen sources are also not limited in any way, and if necessary, known trace nutrient sources and the like may be selected and added, as appropriate. Examples of trace nutrient sources include phosphate ion, alkali metal or alkaline earth metal ions such as calcium ion, sodium ion, and magnesium ion; metal ions of groups VIIB to VIII such as iron, nickel, cobalt and manganese; metal ions of groups IB to IIB such as copper and zinc; as well as various vitamins. The content (addition rate) of each ingredient in a liquid medium is not limited in any way and may be set within a known range as long as it is a concentration at which the microorganisms used in the method of the present invention are not prevented from growing.

Particularly in terms of the productivity of phospholipids, it is preferable to add a phosphate salt. Examples of a phosphate salt to be added include potassium monohydrogen phosphate, potassium dihydrogen phosphate, sodium monohydrogen phosphate, and sodium dihydrogen phosphate. The concentration of a phosphate salt to be added is not limited in any way as long as it is a concentration at which the microorganisms used in the method of the present invention are not prevented from growing, but it is preferably within a range between 3.7 mM and 147 mM, more preferably within a range between 3.7 mM and 44 mM, and particularly preferably within a range between 22 mM and 44 mM. This enables an increase in the phospholipid content within lipid-producing microorganism cells.

The initial pH may be within a range between pH 4 and pH 6, and more preferably it is adjusted to pH 4 to pH 5. The pH during culturing is controlled. In the method of the present invention, the pH during culturing is controlled to maintain between pH 4 and 6. When the *Mortierella sp*. used in the present invention are cultured in a liquid medium, the pH of the culture solution, when not controlling the eM, rises in the late stage of culturing, particularly after depletion of carbon sources. If this rise in pH is prevented and culturing is controlled such that the pH is maintained at a low range between pH 4 and pH 6, preferably between pH 4 and pH 5.5, throughout the culture period from initiation of culturing, the amount of phospholipids accumulated in the cells can be improved to thereby increase the amount of LCPUFA-PL per cell.

The culture temperature is not limited in any way as long as it is a temperature at which the microorganisms used in the method of the present invention are not prevented from growing, but may usually be within a range between 5°C and 40°C, and more preferably within a range between 20°C and 30°C. The culture period is also not limited in any way, but may usually be within a range between 2 and 20 days. For industrial purposes, the culture period is preferably within a range between 3 and 10 days, and more preferably within a range between 3 and 6 days.

In a preferred embodiment, the culture temperature and culture period in the method of the present invention are controlled as follows. First, culturing is carried out within a relatively high temperature range of around 28°C, which is an optimal growth temperature for normal filamentous fungi, for 2 to 20 days, preferably 3 to 10 days, more preferably 3 to 6 days, even more preferably 2 to 4 days, and most preferably 4 days to grow microorganism cells. The cells are then cultured in a temperature range lower than the initial culture temperature to thereby allow an increase in the percentage of polyunsaturated fatty acids (PUFA) in the produced unsaturated fatty acids. The lower temperature range used in this case is preferably from 10°C to 25°C, more preferably from 15°C to 25°C, and in practical application particularly preferably from 20°C to 25°C. The period for such low temperature culturing is not limited in any way, but it is preferably within a range between 4 hours and 18 hours, and more preferably within a range between 12 hours and 18 hours. Such short-term temperature control enables cost savings and an increase in the percentage of LCPUFA in the resulting phospholipids.

Any external treatment may be performed on a medium during culturing, and any known culture technique may be selected, as appropriate, such as aeration-agitation culture, shaking culture or static culture. Particularly in aeration-agitation culture, the productivity of LCPUFA-PL can be improved when the fluidity of a culture solution is increased by agitation. In this case, the required maximum agitation power KLA (= (P/V)^{0.95}·VS^{0.67}) is 8.0 or more, preferably 8.3 or more.

The above optional culture conditions, i.e., including the initial pH, the concentration of a phosphate salt added to the medium, the culture temperature and period, as well as the agitation power, may be used either alone or in combination to achieve increased intracellular accumulation of target LCPUFA-PL.

In the method of the present invention, in order to increase the yield of unsaturated fatty acids including LCPUFA, precursors and/or substrates of the unsaturated fatty acids may be added to the medium. Specific examples of precursors and/or substrates of unsaturated fatty acids include, but are not limited to, hydrocarbons (e.g., hexadecane, octadecane); fatty acids (e.g., oleic acid, linolic acid, docosahexaenoic acid) or salts thereof; fatty acid esters (e.g., ethyl ester, glycerine fatty acid ester, sorbitan fatty acid ester): and oils (e.g., olive oil, soybean oil, rapeseed oil, cottonseed oil, coconut oil, fish oil). These materials may be used either alone or in combination, as appropriate. The above precursors and/or substrates of unsaturated fatty acids may be added in any amount, but they may usually be added in a range between 0.001% and 10% based on the total weight of the medium, and preferably in a range between 0.5% and 10%. Alternatively, these precursors and/or substrates may be used as a sole carbon source to culture lipid-producing microorganisms. In this way, when appropriate substrates are added to the medium, target fatty acids can be efficiently incorporated into phospholipids.

To obtain LCPUFA-PL from the cultured product thus prepared, the method of the present invention has the phospholipid (PL) extraction step as an essential step. In this PL extraction step, any technique may be used for extraction of PL from microorganism cells, but it is preferable to perform extraction with an extraction medium.

In the method of the present invention, the cultured product used for LCPUFA-PL extraction may be a culture solution obtained as described above, which may be used directly or further sterilized. Alternatively, the cultured product may be cultured cells which may be used directly after collection from their culture solution (i.e., used as living cells) or further sterilized. The above culture solution may be obtained either during culturing or upon the completion of culturing. The collected cells may be used directly in pellet form or shaped into any form such as plate, fiber, grain or powder. Although any technique may be used for cell collection, centrifugation with a standard centrifugal separator may be carried out when the cultured cells are small in volume. In a case where the cultured cells are large in volume, they are preferably separated by continuous centrifugation, which may further be combined with filtration through a membrane or the like. Alternatively, the collected cells may be used directly in a wet state or may be used as dried cells by drying these wet cells. Particularly in the present invention, dried cells are preferred for use. This enables efficient extraction of fats. Any technique may be used for drying wet cells, as exemplified by known drying treatment such as air-drying, thermal treatment, vacuum treatment or lyophilization.

In the above extraction with an extraction medium, the extraction medium to be used is not limited in any way and generally includes any extracting solution selected at least from aliphatic organic solvents and water, as well as supercritical carbon dioxide gas. Among these extracting solutions, specific examples of aliphatic organic solvents include saturated hydrocarbons such as hexane and petroleum ether; ketones such as acetone; alcohols such as methanol and ethanol; esters such as ethyl acetate; halogenated hydrocarbons such as chloroform; cyanidated hydrocarbons such as acetonitrile; and ethers such as diethyl ether. These extracting solutions may be used alone or in combination, as appropriate. Among these extracting solutions, an aliphatic organic solvent preferred for efficient extraction of phospholipids is a saturated hydrocarbon, an alcohol, a mixed solvent of a saturated hydrocarbon and an alcohol, or a mixed solvent of a halogenated hydrocarbon and an alcohol. It is preferable to use hexane as a saturated hydrocarbon, to use ethanol as an alcohol, to use a hexane/ethanol mixed solvent as a mixed solvent of a saturated hydrocarbon and an alcohol, and to use a chloroform/methanol mixed solvent as a mixed solvent of a halogenated hydrocarbon and an alcohol. Among these organic solvents, hexane and/or ethanol is/are preferred particularly for use in foods. It should be noted that when a hexane/ethanol mixed solvent or ethanol is used, a small amount of water may be added to this solvent.

The above extraction treatment with an extraction medium can be accomplished either in a batch manner or in a continuous manner. Likewise, the extraction with an extraction medium may be carried out under any conditions, i.e., at an appropriate temperature, using an appropriate amount of the extraction medium and for an appropriate period of time, depending on the type of PL to be extracted and the amount (volume or weight) of microorganism cells. During extraction, the cells are preferably dispersed in the extraction medium and then agitated gently. This enables efficient extraction.

LCPUFA-PL obtained by the method of the present invention may be extracted as a composition dissolved in a liquid fat (e.g., a triglyceride) containing a high concentration of LCPUFA. Examples of such a liquid fat (e.g., a triglyceride) containing a high concentration of LCPUFA include triglycerides, diglycerides, monoglycerides, fatty acids, and fatty acid alcohol esters. LCPUFA as a constituent of the above liquid fat (e.g., a triglyceride) containing a high concentration of LCPUFA is not limited in any way as long as it is a C₂₀ or higher fatty acid with an unsaturated structure having double bonds. LCPUFA-PL obtained by the method of the present invention may also be extracted as a composition containing fat-soluble substances such as sterols, sterol esters, glycolipids, sphingolipids, wax, pigments, carotenoids, tocopherols, etc.

Any technique may be used in the PL purification step, i.e., may be used to purify crude PL obtained in the above PL extraction step. It is possible to use known purification techniques including thin-layer chromatography (TLC), column chromatography, and solvent fractionation (e.g., acetone fractionation). Any carrier may be used for chromatography, with known ones being preferred for use. Likewise, any solvent may be used for solvent fractionation, with known ones being preferred for use.

Phospholipids produced by the method of the present invention contain a long-chain polyunsaturated fatty acid (LCPUFA) as a constituent. LCPUFA as used herein refers to a C₂₀ or higher fatty acid with an unsaturated structure having double bonds.

Phospholipids contained in dried microorganism cells obtained by the method of the present invention are not limited in any way as long as they contain LCPUFA as a constituent (i.e., LCPUFA-PL). All known phospholipids may be included, such as glycerophospholipids, sphingophospholipids and lysophospholipids. Specific examples include phosphatidylcholine (PC), phosphatidylserine (PS), phosphatidylethanolamine (PE), phosphatidylinositol (PI), phosphatidylglycerol (PG), cardiolipin (CL) and the like, for glycerophospholipids; sphingomyelin (SP) and the like, for sphingophospholipids; and lysophosphatidylcholine (LPC), lysophosphatidylserine (LPS), lysophosphatidylethanolamine (LPE), lysophosphatidylinositol (LPI), lysophosphatidylglycerol (LPG), phosphatidic acid and the like, for lysophospholipids. Among these phospholipids, PC, PS, PE, PI, phosphatidic acid and CL are preferred.

LCPUFA contained as a constituent in phospholipids produced by the method of the present invention is not limited in any way as long as it is a C₂₀ or higher fatty acid with an unsaturated structure having double bonds. " Preferred is LCPUFA selected from the group consisting of omega-9 (ω9) polyunsaturated fatty acids, omega-6 (ω6) polyunsaturated fatty acids and omega-3 (ω3), polyunsaturated fatty acids. As used herein, ω9, ω6 and ω3 each mean a series corresponding to the position of a double bond in each unsaturated fatty acid. More specifically, ω9, ω6 and ω3 mean series of unsaturated fatty acids having a double bond at the 9-, 6- and 3-positions, respectively, when carbons are numbered starting from the carbon opposite to the carboxyl group of fatty acid. Specific examples of LCPUFA include 5,8,11-eicosatrienoic acid (Mead acid; 20:3, ω9), 7,10,13-docosatrienoic acid (22:3, ω9), and 4,7,10,13-docosatetraenoic acid (22:4, ω9) for ω9 polyunsaturated fatty acids. Likewise, for ω6 polyunsaturated fatty acids, specific examples include 11,14-eicosadienoic acid (20:2, ω6), 8,11,14-eicosatrienoic acid (dihomo-γ-linolenic acid; 20:3, ω6), 5,8,11,14-eicosatetraenoic acid (arachidonic acid; 20:4, ω6), 13,16-docosadienoic acid (22:2, ω6), 7,10,13,16-docosatetraenoic acid (22:4, ω6), and 4,7,10,13,16-docosapentaenoic acid (22:5, ω6). For ω3 polyunsaturated fatty acids, specific examples include 11,14,17-eicosatrienoic acid (α-linolenic acid; 20:3, ω3), 8,11,14-,17-elcosatetraenoic acid (20:4, ω3), 5,8,11,14,17-eicosapentaenoic acid (20:5, ω3), 7,10,13,16,19-docosapentaenoic acid (22:5, ω3), and 4,7,10,13,16,19-docosahexaenoic acid (22:6, ω3). It should be noted that two numerics sandwiching a colon in parentheses following each LCPUFA name represent the carbon chain length and unsaturation degree of fatty acid, expressed as "(carbon chain length):(number of double bonds)." Among the above examples of LCPUFA, arachidonic acid (ARA) and/or 4,7,10_{,}13,16,19-docosahexaenoic acid (DHA) is/are more preferred for use. These LCPUFAs may be contained either alone or in combination as a constituent of LCPUFA-PL.

In the above LCPUFAs, at least one of the carbon-carbon double bonds (-C=C-) contained in their structure may be a conjugated double bond. This conjugated double bond may be one conjugated with a carbonyl (C=O) group or may be formed between carbon-carbon double bonds adjacent to each other.

The method of the present invention is therefore a method for producing LCPUFA-PL described in more detail above. Likewise, phospholipids produced by the method of the present invention also fall within the scope of the present invention.

Phospholipids produced by the method of the present invention may comprise at least one of the phospholipids listed above or may comprise two or more of them. In the case of crude phospholipids which do not undergo the purification step, they may contain various minor components. Examples of such minor components include lipid components (e.g., fats) other than phospholipids derived from lipid-producing microorganisms.

The composition of LCPUFA-PL obtained by the method of the present invention can be determined by any procedure known to those skilled in the art. For example, qualitative and quantitative analysis for the fatty acid moiety of phospholipids may be accomplished as follows: the fatty acid as a constituent of phospholipids is derived into its methyl ester form by the HCl-methanol method known to those skilled in the art and then evaluated by gas chromatography. Analysis for the hydrophilic moiety of phospholipids may be accomplished by using known separation techniques, such as thin-layer chromatography (TLC), column chromatography, mass spectrometry, solvent fractionation (e.g., acetone fractionation), etc.

Phospholipids obtained from the cultured product according to the method of the present invention are characterized in that branched fatty acids contained as their constituents, such as isomyristic acid (iso14:0) and isopalmitic acid (iso16:0), are in low content. The content of branched fatty acids may be evaluated, for example, as the content of isopalmitic acid. More specifically, "branched fatty acids are in low content" means that the compositional ratio of isopalmitic acid is 5% or less, preferably 0.5% or less, more preferably 0.1% or less, based on the total amount of all fatty acids contained as constituents of all PLs.

In phospholipids comprising LCPUFA-PL obtained by the method of the present invention, the compositional ratio of isopalmitic acid, a branched fatty acid, may.be 5% or less based on the total amount of fatty acids contained as constituents of all phospholipids contained in the cultured product, and the compositional ratio of arachidonic acid may be 13% or more based on the total amount of the fatty acids. Preferably, the compositional ratio of isopalmitic acid may be 0.5% or less or 0.1% or less, and the compositional ratio of arachidonic acid may be 20% or more or 25% or more.

Alternatively, in phospholipids comprising LCPUFA-PL obtained by the method of the present invention, the compositional ratio of isopalmitic acid, a branched fatty acid, may be 5% or less based on the total amount of fatty acids contained as constituents of all phospholipids contained in the cultured product, and the compositional ratio of isopalmitic acid may also be 1/6 or less of the compositional ratio of arachidonic acid based on the total amount of the fatty acids. Preferably, the compositional ratio of isopalmitic acid may be 0.5% or less or 0.1% or less, and the compositional ratio of isopalmitic acid may also be 1/10 or less or 1/20 or less of the compositional ratio of arachidonic acid.

With respect to phospholipids obtained by the method of the present invention, the amount of fatty acids contained as constituents of all phospholipids contained per g of dried cells is not limited in any way, but it is preferably 34.7 mg or more, more preferably 36.3 mg or more, even more preferably 47.7 mg or more, and particularly preferably 65.2 mg or more.

In a case where LCPUFA-PL obtained by the method of the present invention contains arachidonic acid (ARA) as its constituent LCPUFA, the amount of ARA contained as a constituent of all phospholipids contained per g of dried cells is not limited in any way, but it is preferably 12.1 mg or more, more preferably 15.1 mg or more, and even more preferably 19.9 mg or more.

Likewise, in a case where LCPUFA-PL obtained by the method of the present invention contains dihomo-y-linblenic acid (DGLA) as its constituent LCPUFA, the amount of DGLA contained as a constituent of all phospholipids contained per g of dried cells is not limited in any way, but it is preferably 1.5 mg or more, more preferably 1.8 mg or more, and even more preferably 2.0 mg or more.

In a case where LCPUFA-PL obtained by the method of the present invention is phosphatidylcholine (PC), the amount of fatty acids contained as constituents of all PCs contained per g of dried cells is not limited in any way, but it is 5.99 mg or more, preferably 10.0 mg or more, more preferably 15.0 mg or more, and even more preferably 20.0 mg or more. Likewise, in a case where LCPUFA-PL obtained by the method of the present invention is phosphatidylethanolamine (PE), the amount of fatty acids contained as constituents of all PEs contained per g of dried cells is not limited in any way, but it is 7.62 mg or more, preferably 10.0 mg or more, more preferably 15.0 mg or more, and even more preferably 20.0 mg or more. Further, in a case where LCPUFA-PL obtained by the method of the present invention is phosphatidylserine (PS), the amount of fatty acids contained as constituents of all PSs contained per g of dried cells is not limited in any way, but it is 1.09 mg or more, preferably 2.0 mg or more, and more preferably 3.0 mg or more.

In a case where LCPUFA-PL obtained by the method of the present invention comprises phosphatidylcholine containing arachidonic acid (ARA) as a constituent (ARA-PC), the amount of arachidonic acid contained as a constituent of all phosphatidylcholines is not limited in any way, but it is 1.0 mg or more, preferably 1.08 mg or more, more preferably 4.0 mg or more, and even more preferably 5.0 mg or more, per g of dried cells. Likewise, in a case where LCPUFA-PL obtained by the method of the present invention comprises phosphatidylethanolamine containing ARA as a constituent (ARA-PE), the amount of arachidonic acid contained as a constituent of all phosphatidylethanolamines is not limited in any way, but it is 0.5 mg or more, preferably 1.35 mg or more, more preferably 2.0 mg or more, even more preferably 3.0 mg or more, and particularly preferably 5.0 mg or more, per g of dried cells. Further, in a case where LCPUFA-PL obtained by the method of the present invention comprises phosphatidylserine containing ARA as a constituent (ARA-PS), the amount of arachidonic acid contained as a constituent of all phosphatidylserines is not limited in any way, but it is 0.05 mg or more, preferably 0.06 mg or more, more preferably 0.15 mg or more, even more preferably 0.3 mg or more, and particularly preferably 0.4 mg or more, per g of dried cells.

In a case where LCPUFA-PL obtained by the method of the present invention comprises phosphatidylcholine containing dihomo-γ-linolenic acid (DGLA) as a constituent (DGLA-PC), the amount of DGLA contained as a constituent of all phosphatidylcholines is not limited in any way, but it is 0.11 mg or more, preferably 0.3 mg or more, and more preferably 0.6 mg or more, per g of dried cells. In a case where LCPUFA-PL obtained by the method of the present' invention comprises phosphatidylethanolamine containing DGLA as a constituent (DGLA-PE), the amount of DGLA contained as a constituent of all phosphatidylethanolamines is not limited in any way, but it is 0.1 mg or more, preferably 0.3 mg or more, and more preferably 0.5 mg or more, per g of dried cells.

The cultured product obtained by the method of the present invention contains, in addition to LCPUFA-PL, triglycerides containing LCPUFA as a constituent, and their percentage is not limited in any way. However, the cultured product prepared by the method of the present invention has an increased content of LCPUFA-PL when compared to the cultured products prepared by other culturing techniques. Whether the content of LCPUFA-PL in the cultured product is increased can be confirmed, for example, by evaluating the ratio between arachidonic acid contained as a constituent of phospholipids (ARA-PL) and arachidonic acid contained as a constituent of triglycerides (ARA-TG). In the cultured product obtained by the method of the present invention, for example, the ratio between ARA-PL and ARA-TG (ARA-PL/ARA-TG) is 0.2 or more, preferably 0.84 or more, more preferably 1.05 or more, and even more preferably 1.70 or more.

When the method of the present invention is used to culture *Mortierella sp.* that are capable of producing phospholipids containing LCPUFA as a constituent (LCPUFA-PL), a large amount of LCPUFA-PL can be accumulated in the above cells for a short time. Moreover, the phospholipids in the cells produced by the method of the present invention have a low content of branched fatty acids. For this reason, these cells, when used as a supply source for LCPUFA-PL extraction, enable efficient and stable provision of LCPUFA-PL as a high quality industrial product available for use in foods and animal feed with more safety, although LCPUFA-PL has been obtained in a small amount from limited sources, so that its supply has been unstable and its quality has not always been consistent.

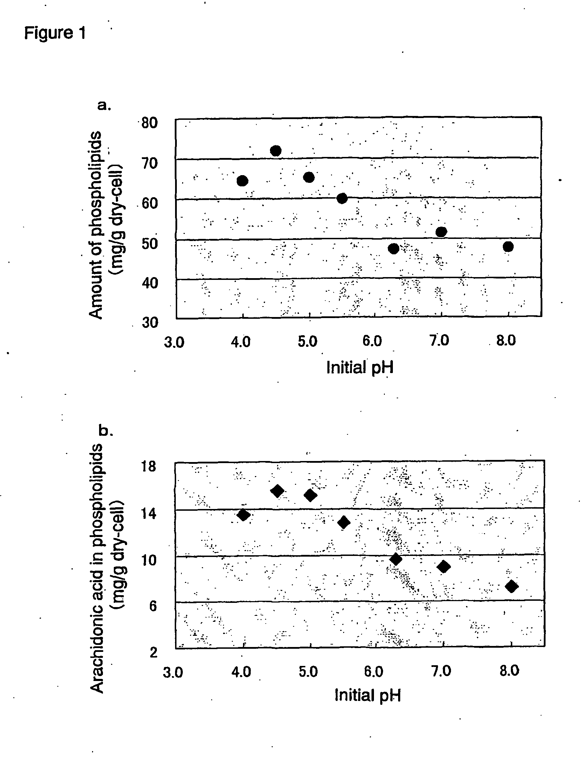

Figure 1a is a graph showing the correlation between the initial pH and the amount of phospholipids extracted. Figure 1b is a graph showing the correlation between the initial pH and the amount of arachidonic acid in the extracted phospholipids.

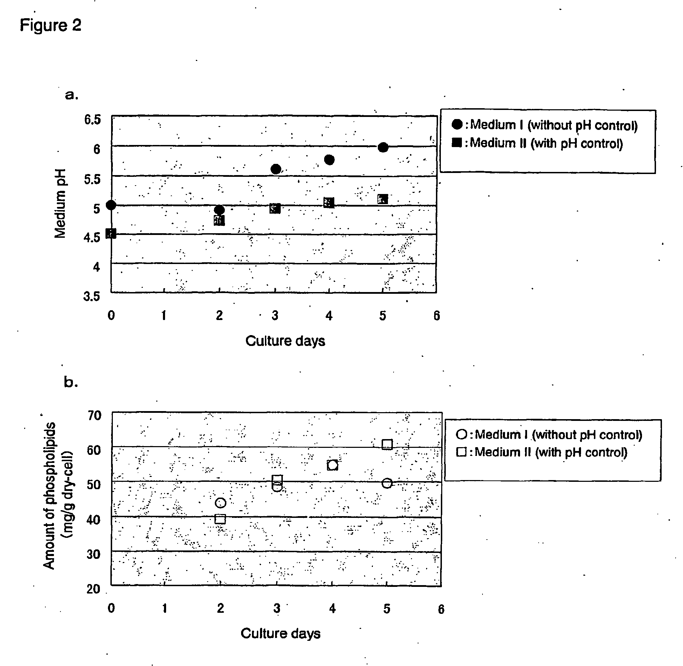

Figure 2a is a graph showing pH changes during culturing in Medium I and Medium II. Solid circle represents Medium I (without pH control) and solid square represent Medium II (with pH control). Figure 2b is a graph showing the correlation between culture days, and the amount of phospholipids extracted. Open circle represents Medium I (without pH control) and open square represents Medium II (with pH control).

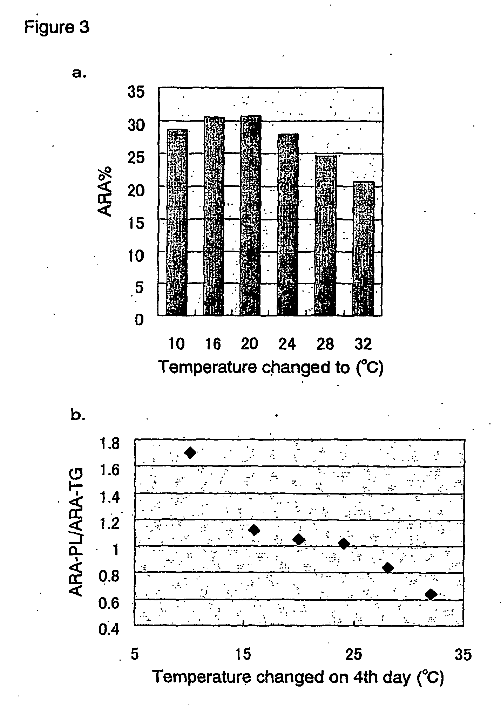

Figure 3a is a graph showing the percentage of arachidonic acid (ARA) relative to all fatty acids in the phospholipid fraction after the temperature was changed on the fourth day of culturing. Figure 3b is a graph showing the ratio of arachidonic acid present in the phospholipid fraction to arachidonic acid present in the triglyceride fraction after the temperature was changed on the fourth day of culturing

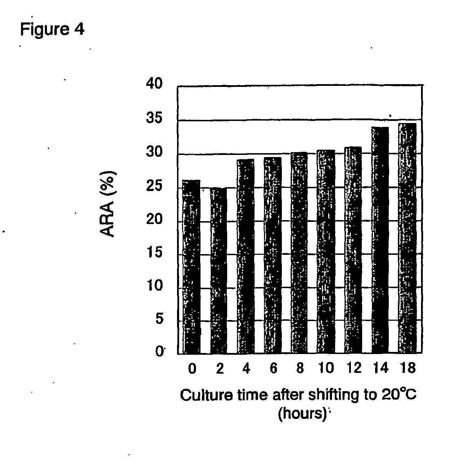

Figure 4 is a graph showing the percentage of arachidonic acid (ARA) relative to all fatty acids in the phospholipid fraction, as expressed versus the culture time, after the temperature was shifted to 20°C on the fourth day of culturing.

### EXAMPLES

The present invention will now be described in more detail by way of the following examples and comparative examples, which are not intended to limit the scope of the invention. In addition, the term "phospholipid" appearing in the following examples and comparative examples is defined as a fraction that is not eluted with chloroform washing on a silica gel column, but is collected by the subsequent methanol elution, or defined as a fraction that does not move at all from the spotted position in silica gel thin-layer chromatography (TLC) using hexane:diethyl ether = 7:3 as a developing solvent.

### Example 1: Productivity of branched fatty acid (isopalmitic acid (iso16:0))

A medium (pH 6.3, 20 mL) containing 1% yeast extract and 4% glucose was prepared in 100 mL Meyer's flasks and sterilized at 120°C for 20 minutes. Two strains belonging to *Conidiobolus sp.,* i.e., *Conidiobolus thromoboides* CBS183.60 and *Conidiobolus nanodes* CBS154.56, as well as Strains A to E belonging to *Mortierella sp.* shown in Table 1 were respectively inoculated in an amount of one platinum loop into the medium, followed by culturing for 7 days under conditions of 120 rpm reciprocal shaking at a temperature of 28°C. After the completion of culturing, the cells were collected by filtration, sufficiently washed with water and then lyophilized overnight to obtain dried cells for each case.

A mixed solvent of chloroform/methanol = 2:1 (4 mL) was added to the resulting dried cells and each mixture was gently agitated at 70°C for 1 hour to collect the organic solvent layer, followed by re-addition of the above mixed solvent (4 mL) to collect all the organic solvent layers. This organic solvent was distilled off with a centrifugal evaporator to obtain a total lipid fraction. The resulting total lipid fraction was fractionated by silica gel thin-' layer chromatography (TLC) into triglyceride (TG) and phospholipid (PL) fractions. The developing solvent used was a mixed solvent of hexane:ethyl ether = 7:3 (v/v). Both fractions were each collected by scraping and derived into a methyl ester form by the HCl-methanol method, followed by qualitative and quantitative analysis of fatty acids by gas chromatography. Pentadecanoic acid was used as an internal standard.

The results obtained are shown in Table 2. It should be noted that all weights in Table 2 are those per g of dried cells.

**Table 1**

| | Cultured strains of *Mortierella sp.* |
|---|---|
| Dried cell A | A: *Mortierella alpina* 1S-4 AKU3998 |
| Dried cell B | B: *Mortierella schmuckeri* NRRL2761 |
| Dried cell C | C: *Mortierella alpina* CBS224.37 |
| Dried cell D | D: *Mortieralla beljakovae* CBS601.68 |
| Dried cell E | E: *Mortierella elongata* CBS125.71 |

**Table 2**

| Dried cells | Extracted fraction | Extracted lipid (as fatty acid) (mg) | Percentages of ARA and iso16:0 relative to all fatty acids in each fraction (%) | | "iso 16:0/ARA" ratio in each fraction | ARA distribution "ARA-PL/ARA-TG" ratio |
|---|---|---|---|---|---|---|
| | | | ARA | iso16:0 | | |
| *Conidiobolus thromobides* | PL | 43.3 | 32.9 | 5.81 | 0.18 | 1.16 |
| | TG | 161.8 | 7.58 | 6.44 | 0.85 | |
| *Conidiobolus nanodes* | PL | 33.5 | 22.8 | 6.96 | 0.30 | 0.95 |
| | TG | 141.5 | 5.69 | 14.44 | 2.54 | |
| A | PL | 20.5 | 26.7 | 0' | 0 | 0.30 |
| | TG | 114.6 | 15.9 | 0.09 | 0.01 | |
| B | PL | 36.3 | 29.2 | 0 | 0 | 1.49 |
| | TG | 284 | 2.5 | 0.02 | 0.01 | |
| C | PL | 36.8 | 26.1 | 0 | 0 | 4.41 |
| | TG | 10.9 | 20.0 | 0 | 0 | |
| D | PL | 47.8 | 12.9 | 0.33 | 0.03 | 1.40 |
| | TG | 122 | 3.6 | 0.36 | 0.10 | |
| E | PL | 45.3 | 18.0 | 0.11 | 0.01 | 0.84 |
| | TG | 116.6 | 8.4 | 0.24 | 0.03 | |

| | | | | | | |
|---|---|---|---|---|---|---|
| *The abbreviations in the table have the following meanings: PL: phospholipid, TG: triglyceride, ARA: arachidonic acid, iso16:0: Isopaimitic acid, ARA-PL: arachidonic acid contained as a constituent of phospholipids, ARA-TG: arachidonic acid contained as a constituent of triglycerides. | | | | | | |

The *Mortierella sp.* were confirmed to accumulate phospholipids containing a high percentage of arachidonic acid and having a low content of branched fatty acids when compared to the *Conidiobolus sp.*

### Example 2: Composition of LCPUFA-containing phospholipids in Mortierella sp.

A medium (pH 6.3, 4 mL) containing 1% yeast extract and 4% glucose was prepared in 20 mL Meyer's flasks and sterilized at 120°C for 20 minutes. Strains A, B, D and E shown in Table 1 were respectively inoculated in an amount of one platinum loop into the medium, followed by culturing for 7 days under conditions of 120 rpm reciprocal shaking at a temperature of 28°C to obtain dried cells A', B', D' and E'. Likewise, a medium (pH 5.0, 4 ml) containing 1% soy flour, 1.5% glucose and 0.3% KH₂PO₄ was prepared in a 20 ml Meyer's flask and then inoculated with Strain C shown in Table 1, followed by culturing in the same manner as shown above to obtain dried cell C'.

Each total lipid fraction obtained in the same manner as shown in Example 1 was subjected to TLC (the developing solvent used was chlorbform:methanol:acetic acid:water = 50:37.5:3.5:2 (v/v/v/v)) and fractionated into the respective phospholipid fractions, i.e., phosphatidylcholine (PC), phosphatidylserine (PS), phosphatidylethanolamine (PE), phosphatidylinositol (PI) and cardiolipin (CL) fractions, followed by qualitative and quantitative analysis of fatty acids in the same manner as shown in Example 1.

The results obtained are shown in Table 3. It should be noted that all weights in Table 3 are those per g of dried cells.

**Table 3**

| Dried cells | Phospholipid fraction | Extracted lipid (as fatty acid) (mg) | Amount of LCPUFA relative to all fatty acids in each fraction | |
|---|---|---|---|---|
| | | | ARA (mg) | DGLA (mg) |
| A' | PC | 5.99 | 1.08 | 0.11 |
| | PE | 7.62 | 1.35 | 0.19 |
| | PS | 1.09 | 0.06 | ND |
| | PI | 1.42 | 0.02 | 0.02 |
| | CL | 1.36 | 0.05 | ND |
| B' | PC | 12.4 | 5.37 | 0.16 |
| | PE | 12.1 | 2.81 | 0.10 |
| | PS | 3.56 | 0.70 | 0.01 |
| | PI | 3.19 | 0.09 | 0.06 |
| | CL | 2.39 | 0.12 | ND |
| C' | PC | 20.7 | 6.53 | 0.65 |
| | PE | 26.4 | 5.67 | 0.75 |
| | PS | 1.95 | 0.36 | 0.04 |
| | PI | 5.32 | 0.25 | 0.26 |
| | CL | 2.40 | 0.21 | 0.16 |
| D' | PC | 16.2 | 4.42 | 0.87 |
| | PE | 19.2 | 2.43 | 0.56 |
| | PS | 2.20 | 0.18 | 0.03 |
| | PI | 4.00 | 0.05 | 0.12 |
| | CL | 3.90 | 0.22 | 0.17 |
| E' | PC | 14.1 | 4.55 | 0.33 |
| | PE | 16.6 | 3.27 | 0.36 |
| | PS | 3.30 | 0.43 | 0.03 |
| | PI | 4.00 | 0.13 | 0.16 |
| | CL | 3.50 | 0.22 | 0.16 |

| | | | | |
|---|---|---|---|---|
| *ND: non-detectable by TLC *The abbreviations in the table have the following meanings: LCPUFA: long-chain polyunsaturated fatty acid, ARA: arachidonic acid, DGLA: dihomo-γ-linolenic acid. | | | | |

### Example 3: Culturing in the presence of phosphate salt

Media (pH 6.3, 4 mL) containing 1% soy flour, 3% glucose and KH₂PO₄ at 6 different concentrations shown in Table 4 were prepared in 20 mL Meyer's flasks and sterilized at 120°C for 20 minutes. A spore suspension of *Mortierella schmuckeri* NRRL2761 or *Mortierella alpina* CBS224.37 was inoculated into the above media, followed by culturing for 5 days under conditions of 120 rpm reciprocal shaking at a temperature of 28°C. After the completion of culturing, all lipids were extracted from the lyophilized cells in the same manner as shown in Example 1 and fractionated by Sep-Pak Plus column chromatography into triglyceride (TG) and phospholipid (PL) fractions. The elution solvent used was chloroform for triglyceride elution and methanol for phospholipid elution, and these organic solvents were distilled off with a centrifugal evaporator to obtain each fraction. After fractionation, each fraction was derived into a methyl ester form by the HCl-methanol method, followed by qualitative and quantitative analysis of fatty acids by gas chromatography. Pentadecanoic acid was used as an internal standard.

The results obtained are shown in Table 4. It should be noted that all weights in Table 4 are those per g of dried cells.

**Table 4**

| Strain | KH₂PO₄ concentration | Extracted phospholipid (mg) (as fatty acid) | Amount of LCPUFA in phospholipid (mg) | | ARA distribution "ARA-PL/ ARA-TG" ratio |
|---|---|---|---|---|---|
| | | | ARA | DGLA | |
| *Mortierella schmuckeri* | 0% | 21.8 | 4.9 | 0.4 | 0.41 |
| | 0.05% (3.7 mM) | 33.4 | 7.7 | 1.0 | 0.54 |
| | 0.3% (22 mM) | 37.3 | 7.9 | 1.1 | 0.64 |
| | 0.4% (29 mM) | 25.2 | 7.5 | 0.9 | 0.60 |
| | 0.6% (44 mM) | 36.8 | 8.0 | 1.0 | 0.68 |
| | 2% (147 mM) | 24.6 | 5.0 | 0.5 | 0.62 |
| *Mortierella alpina* | 0% | 42.3 | 8.8 | 1.7 | 0.30 |
| | 0.05% (3.7 mM) | 58.3 | 13.3 | 1.9 | 0.82 |
| | 0.3% (22 mM) | 57.8 | 13.1 | 1.9 | 0.79 |
| | 0.4% (29 mM) | 55.8 | 12.1 | 1.8 | 0.85 |
| | 0.6% (44 m M) | 49.8 | 10.4 | 1.5 | 1.05 |
| | 2% (147 mM) | 37.2 | 6.5 | 0.9 | 1.64 |

| | | | | | |
|---|---|---|---|---|---|
| *The abbreviations in the table have the following meanings: LCPUFA: long-chain polyunsaturated fatty acid, ARA: arachidonic acid, DGLA: dihomo-γ-linolenic acid, ARA-PL: arachidonic acid contained as a constituent of phospholipids, ARA-TG: arachidonic acid contained as a constituent of triglycerides. | | | | | |

The results confirmed that a phosphate salt, when added to the medium, increased the amount of phospholipids per cell and the amount of LCPUFA in phospholipids, and that the effect of phosphate salt addition was more effective in phospholipids than in triglycerides.

### Example 4: Culturing with low initial pH

The initial pH of a medium containing 1% soy flour, 1.5% glucose and 0.3% KH₂PO₄ was adjusted to 7 levels (i.e., pH 4.0, pH 4.5, pH 5.0, pH 5.5, pH 6.3, pH 7.0 and pH 8.0), and 4 mL of the medium was introduced into a 20 mL Meyer's flask and sterilized at 120°C for 20 minutes. A spore suspension of *Mortierella alpina* CBS224.37 was inoculated into the above medium, followed by culturing for 5 days under conditions of 120 rpm reciprocal shaking at a temperature of 28°C. After the completion of culturing, all lipids were extracted from the lyophilized cells in the same manner as shown in Example 3 and fractionated by Sep-Pak Plus column chromatography into triglyceride (TG) and phospholipid (PL) fractions, followed by qualitative and quantitative analysis of fatty acids.

The results obtained are shown in Table 5 and Figure 1. It should be noted that all weights in Table 5 are those per g of dried cells.

**Table 5**

| Initial pH | Extracted phospholipid as fatty acid (mg) | Amount of LCPUFA in phospholipid fraction (mg) | | Percentage of LCPUFA in phospholipid fraction (%) | |
|---|---|---|---|---|---|
| | | ARA | DGLA | ARA | DGLA |
| 4.0 | 64.6 | 13.5 | 2.0 | 21.0 | 3.1 |
| 4.5 | 71.9 | 15.5 | 2.3 | 21.6 | 3.2 |
| 5.0 | 65.2 | 15.1 | 2.4 | 23.2 | 3.6 |
| 5.5 | 59.8 | 12.8 | 2.0 | 21.4 | 3.3 |
| 6.3 | 47.1 | 9.6 | 1.4 | 20.4 | 2.9 |
| 7.0 | 51.4 | 8.9 | 1.2 | 17.3 | 2.4 |
| 8.0 | 47.5 | 7.2 | 1.1 | 15.2 | 2.3 |

| | | | | | |
|---|---|---|---|---|---|
| *The abbreviations in the table have the following meanings: LCPUFA: long-chain polyunsaturated fatty acid, ARA: arachidonic acid, DGLA4: dihomo-γ-linolenic acid. | | | | | |

When lowering the initial pH, an increase was observed in the percentage of LCPUFA in phospholipids, and in particular, significant increases were observed in the amount of phospholipids per cell and the amount of LCPUFA in phospholipids.

### Example 5: Low pH control in late stage of culturing

A medium (pH 5.0, 5 L) containing 1.5% soy flour, 2% glucose and 0.3% KH₂PO₄ was introduced into a 10 L jar fermentor and sterilized at 120°C for 20 minutes (Medium I). Likewise, a medium (pH 4.5, 5 L) containing 1.5% soy flour and 2% glucose was introduced into a 10 L jar fermentor and sterilized in the same manner at 120°C for 20 minutes (Medium II). *Mortieralla alpina* CBS224.37 was inoculated, followed by aeration-agitation culture at an aeration volume of 1 vvm for 6 days. The agitation was set to 300 rpm at the initiation of culturing and increased to 500 rpm from the second day of culturing, while the culture temperature was set to 28°C at the initiation of culturing and lowered to 20°C from the third day of culturing. Glucose was added in an amount of 1.0% on the first day of culturing and 0.75% on the second day of culturing. Moreover, only Medium II was controlled with 0.5 N H₂SO₄ such that the medium pH was maintained at or below 5.0 throughout the 6 days of culturing. After the completion of culturing, the same procedure as shown in Example 3 was repeated to perform extraction of all lipids, fractionation into triglyceride (TG) and phospholipid (PL) fractions, and qualitative and quantitative analysis of fatty acids.

The results obtained on the fifth day of culturing are shown in Table 6. It should be noted that all weights in Table 6 are those per g of dried cells. Also, Figure 2a shows pH changes during culturing and Figure 2b shows the effect of pH control on changes in the amount of phospholipids extracted.

**Table 6**

| Sampling date | Medium pH | | Extracted phospholipid (as fatty acid) (mg) | | Amount of LCPUFA in phospholipid fraction (mg) | | | | PL productivity per medium (g/L) "II/I" ratio |
|---|---|---|---|---|---|---|---|---|---|
| | | | | | ARA | | DGLA | | |
| | I | II | I | II | I | II | I | II | |
| Fifth day | 6.0 | 5.1 | 49.7 | 60.7 | 19.1 | 22.7 | 1.21 | 1.60 | 1.40 |

| | | | | | | | | | |
|---|---|---|---|---|---|---|---|---|---|
| *The abbreviations in the table have the following meanings: LCPUFA: long-chain polyunsaturated fatty acid, ARA: arachidonic acid, DGLA: dihomo-γ-linolenic acid, PL: phospholipid. | | | | | | | | | |

The culturing procedure in which the pH rise in the late stage of culturing was prevented (Medium II) provided better results for the amount of LCPUFA per dried cell, particularly the productivity per medium, thus indicating that pH control for prevention of pH rise in the late stage of culturing was effective in a 10 L jar fermentor.

### Example 6: Agitation control

A medium (pH 5.0, 5 L) containing 1.0% soy flour, 2% glucose and 0.3% KH₂PO₄ was introduced into a 10 L jar fermentor and sterilized at 120°C for 20 minutes. *Mortierella alpina* CBS224.37 was inoculated, followed by aeration-agitation culture at a culture temperature of 28°C and an aeration volume of 1 vvm for 7 days. The agitation was varied at 4 levels as indicated in Table 7 on the second day of culturing, and culturing was further continued. On the second day, 6.5% glucose was added. After the completion of culturing, the same procedure as shown in Example 3 was repeated to perform extraction of all lipids, fractionation into triglyceride (TG) and phospholipid (PL) fractions, and qualitative and quantitative analysis of fatty acids.

The results obtained on the fourth day of culturing are shown in Table 7. It should be noted that all weights in Table 7 are those per g of dried cells. It should also be noted that "agitation parameter KLA" appearing in Table 7 means the required maximum agitation power KLA (= (P/V)^{0.95} Vs^{0.67})[(W/m³)^{0.95}(m/sec)^{0.67}], which is defined from the required agitation power per unit volume P/V (W/m³) and the aeration linear velocity parameter Vs (m/sec).

**Table 7**

| Agitation parameter KLA on and after the second day of culturing | Percentage of ARA relative to all fatty acids In phospholipid fraction (%) | Amount of ARA In phospholipid fraction (mg) |
|---|---|---|
| 2.60 | 20.6 | 10.7 |
| 8.27 | 34.1 | 17.1 |
| 12.8 | 36.2 | 18.5 |
| 18.8 | 39.9 | 20.2 |

| | | |
|---|---|---|
| *All of the jars were cultured at KLA=8.27 on the first day. ARA means arachidonic acid. | | |

When KLA was increased to improve the fluidity of the culture solution, it was possible to increase both the percentage of arachidonic acid in the phospholipid fraction and the amount of arachidonic acid per dried cell.

### Example 7: Culturing at low temperature

A medium (pH 5.0, 20 mL) containing 1% soy flour, 1.5% glucose and 0.3% KH₂PO₄ was introduced into a 100 mL Meyer's flask and sterilized at 120°C for 20 minutes. A spore suspension of *Mortierella alpina* CBS224.37 was inoculated into the above medium and cultured with 120 rpm reciprocal shaking at a temperature of 28°C for 4 days. The culture temperature was then varied at 6 levels (i.e., 10°C, 16°C, 20°C, 24°C, 28°C and 32°C) and culturing was further continued with 120 rpm reciprocal shaking for 18 hours. After the completion of culturing, the same procedure as shown in Example 3 was repeated to perform extraction of all lipids, fractionation into triglyceride (TG) and phospholipid (PL) fractions, and qualitative and quantitative analysis of fatty acids. The results obtained are shown in Table 8 and Figure 3.

Likewise, the same medium as mentioned above was inoculated with a spore suspension of *Mortierella alpina* CBS224.37 and cultured with 120 rpm reciprocal shaking at a temperature of 28°C for 4 days. The culture temperature was then lowered to 20°C and culturing was further continued for 2, 4, 8, 12, 14 or 18 hours. After the completion of culturing, the same procedure as shown in Example 3 was repeated to perform extraction of all lipids, fractionation into triglyceride (TG) and phospholipid (PL) fractions, and qualitative and quantitative analysis of fatty acids. The results obtained are shown in Table 9 and Figure 4.

It should be noted that all weights in Tables 8 and 9 are those per g of dried cells.

**Table 8**

| Temperature changed on the fourth day (°C) | Percentage of ARA relative to all fatty acids in phospholipid fraction (%) | Amount of ARA in phospholipid fraction (mg) | ARA distribution "ARA-PL/ARA-TG" ratio |
|---|---|---|---|
| 32 | 20.8 | 11.5 | 0.64 |
| 28 | 24.6 | 13.4 | 0.84 |
| 24 | 27.9 | 16.4 | 1.02 |
| 20 | 30.7 | 17.5 | 1.05 |
| 16 | 30.6 | 17.0 | 1.12 |
| 10 | 28.7 | 15.3 | 1.70 |

| | | | |
|---|---|---|---|
| *The abbreviations in the table have the following meanings: ARA: arachidonic acid, ARA-PL arachidonic acid contained as a constituent of phospholipids, ARA-TG: arachidonic acid contained as a constituent of triglycerides | | | |

**Table 9**

| Culture time at 20°C (hours) | Percentage of ARA relative to all fatty acids In phospholipid fraction (%) | Amount of ARA in phospholipid fraction (mg) |
|---|---|---|
| 0 | 22.9 | 9.6 |
| 2 | 22.1 | 9.0 |
| 4 | 26.2 | 11.5 |
| 6 | 26.4 | 11.5 |
| 8 | 27.2 | 12.1 |
| 10 | 27.5 | 12.1 |
| 12 | 28.0 | 12.6 |
| 14 | 30.9 | 16.1 |
| 18 | 31.4 | 15.9 |

| | | |
|---|---|---|
| *ARA means arachidonic acid. | | |

When shifting to a low temperature during culturing, significant increases were observed in both the percentage of arachidonic acid in the phospholipid fraction and the amount of arachidonic acid per dried cell (Table 8, Figure 3a). These effects appeared even in 4 hr culturing at low temperature and hence were confirmed to become effective within a very short time (Table 9, Figure 4). Moreover, the effect of increasing arachidonic acid was higher in the phospholipid fraction than in the triglyceride fraction, thus enabling intracellular accumulation of fats having a high "ARA-PL/ARA-TG" ratio (Table 8, Figure 3b).

## Claims

1. A method for producing a phospholipid containing a C₂₀ or higher long-chain polyunsaturated fatty acid as a constituent (LCPUFA-PL), which comprises:
(i) culturing a microorganism belonging to *Mortierella sp*. wherein the culture is controlled to maintain between pH 4 and 6, thereby allowing intracellular accumulation of LCPUFA-PL in the microorganism; and
(ii) obtaining LCPUFA-PL from the cultured product of the microorganism, wherein the compositional ratio of isopalmitic acid, a branched fatty acid, is 5% or less based on the total amount of fatty acids contained as constituents of all phospholipids (PL) contained in the cultured product.

2. The method according to claim 1, wherein the microorganism belonging to *Mortierella sp*. is a microorganism of the *Mortierella* subgenus.

3. The method according to claim 2, wherein the microorganism belonging to *Mortierella sp*. is *Mortierella alpina*.

4. The method according to any one of claims 1 to 3, wherein the culturing further comprises culturing under conditions including an initial pH of 4 to 6.

5. The method according to any one of claims 1 to 4, wherein the culturing further comprises culturing in a medium containing 3.7 mM to 147 mM of a phosphate salt.

6. The method according to any one of claims 1 to 5, wherein the culturing further comprises culturing while agitating at a required maximum agitation power (KLA = (P/V)^{0.95}·Vs^{0.67}) of 8.3 or more.

7. The method according to any one of claims 1 to 6, wherein the culturing further comprises culturing at 10°C to 25°C for 4 to 18 hours after growing the cells.

8. The method according to any one of claims 1 to 6, wherein the culturing further comprises culturing at 10°C to 25°C for 4 to 18 hours after culturing at 28°C for 2 to 20 days.

9. The method according to any one of claims 1 to 8, wherein the cultured product is a culture solution or a sterilized culture solution, or comprises cultured cells collected therefrom or dried cells thereof.

10. The method according to any one of claims 1 to 9, wherein LCPUFA-PL comprises at least one glycerophospholipid selected from the group consisting of phosphatidylcholine, phosphatidylserine, phosphatidylethanolamine, phosphatidylinositol, phosphatidic acid and cardiolipin.

11. The method according to any one of claims 1 to 10, which is used for production of LCPUFA-PL containing, as a constituent, a long-chain polyunsaturated fatty acid (LCPUFA) selected from the group consisting of omoga-6 polyunsaturated fatty acids, omega-3 polyunsaturated fatty acids and omega-9 polyunsaturated fatty acids.

12. The method according to claim 11, wherein the omega-6 polyunsaturated fatty acids are one or more fatty acids selected from the group consisting of 11,14-eicosadienoic acid (20:2, ω6), 8,11,14-eicosatrienoic acid (dihomo-γ-linolenic acid; 20:3, ω6), 5,8,11,14-eicosatetraenoic acid (arachidonic acid: 20:4, ω6), 13,16-docosadienoic acid (22:2, ω6), 7,10,13,16-docosatetraenoic acid (22:4, ω6) and 4,7,10,13,16-docosapentaenoic acid (22:5, ω6).

13. The method according to claim 11, wherein the omega-3 polyunsaturated fatty acids are one or more fatty acids selected from the group consisting of 11,14,17-eicosatrienoic acid (α-linolenic acid; 20:3, ω3), 8,11,14,17-eicosatetraenoic acid (20:4, ω3), 5,8,11,14.17-eicosapentaenoic acid (20:5, ω3), 7,10,13,16,19-docosapentaenoic acid (22:5, ω3) and 4,7,10,13,16,19-docosahexaenoio acid (22:6, ω3).

14. The method according to claim 11, wherein the omega-9 polyunsaturated fatty acids are one or more fatty acids selected from the group consisting of 5,8,11-eicosatrienoic acid (Mead acid; 20:3, ω9), 7,10,13-docosatrienoic acid (22:3, ω9) and 4,7,10,13-docosatetraenoic acid (22:4, ω9).

15. The method according to any one of claims 1 to 10, which is used for production of LCPUFA-PL containing, as a constituent, LCPUFA selected from the group consisting of arachidonic acid (5,8,11,14-eicosatetraenoic acid) and docosahexaenoic acid (4,7,10,13,16,19-docosahexaenoic acid).

16. The method according to any one of claims 1 to 10, which is used for production of LCPUFA-PL containing, as a constituent, LCPUFA having at least one conjugated double bond in its molecule.

17. The method according to any one of claims 1 to 10, wherein the compositional ratio of isopalmitic acid, a branched fatty acid, is 5% or less based on the total amount of fatty acids contained as constituents of all phospholipids (PL) contained in the cultured product, and the compositional ratio of arachidonic acid is 13% or more based on the total amount of the fatty acids.

18. The method according to any one of claims 1 to 10, wherein the compositional ratio of isopalmitic acid, a branched fatty acid, is 5% or less based on the total amount of fatty acids contained as constituents of all phospholipids (PL) contained in the cultured product, and the compositional ratio of isopalmitic acid is also 1/6 or less of the compositional ratio of arachidonic acid based on the total amount of the fatty acids.

19. The method according to any one of claims 1 to 10, wherein LCPUFA-PL comprises a phospholipid containing arachidonic acid, and the amount of arachidonic acid in all phospholipids contained in the cultured product is 11.8 mg or more per g of dried cells.

20. The method according to any one of claims 1 to 10, wherein LCPUFA-PL comprises a phospholipid containing dihomo-γ-linolenic acid (DGLA), and the amount of DGLA in all phospholipids contained in the cultured product is 1.5 mg or more per g of dried cells.

21. The method according to any one of claims 1 to 9, wherein LCPUFA-PL comprises a phosphatidylcholine containing arachidonic acid, and the amount of arachidonic acid contained as a constituent of all phosphatidylcholines contained in the cultured product is 1.0 mg or more per g of dried cells.

22. The method according to any one of claims 1 to 9, wherein LCPUFA-PL comprises a phosphatidylethanolamine containing arachidonic acid, and the amount of arachidonic acid contained as a constituent of all phosphatidylethanolamines contained in the cultured product is 0.5 mg or more per g of dried cells.

23. The method according to any one of claims 1 to 9, wherein LCPUFA-PL comprises a phosphatidylserine containing arachidonic acid, and the amount of arachidonic acid contained as a constituent of all phosphatidylserines contained in the cultured product is 0.05 mg or more per g of dried cells.

24. The method according to any one of claims 1 to 10, wherein the cultured product contains a phospholipid containing arachidonic acid as a constituent and a triglyceride containing arachidonic acid as a constituent, wherein the amount of arachidonic acid present as a constituent of the phospholipid is in a ratio of 0.2 or more relative to the amount of arachidonic acid present as a constituent of the triglyceride.

## Patentansprüche

1. Ein Verfahren zur Herstellung eines Phospholipids, das eine langkettige mehrfach ungesättigte Fettsäure mit 20 oder mehr Kohlenstoffatomen als einen Bestandteil (LCPUFA-PL) enthält, welches umfasst:
(i) Kultivieren eines zu *Mortierella sp.* gehörenden Mikroorganismus, wobei die Kultur so gesteuert wird, dass der pH zwischen 4 und 6 gehalten wird, um **dadurch** die intrazelluläre Anhäufung von LCPUFA-PL in dem Mikroorganismus zu ermöglichen; und
(ii) Gewinnen von LCPUFA-PL aus dem kultivierten Produkt des Mikroorganismus, wobei das Zusammensetzungsverhältnis von Isopalmitinsäure, einer verzweigten Fettsäure, 5% oder weniger, bezogen auf die Gesamtmenge an Fettsäuren, die als Bestandteile aller in dem kultivierten Produkt enthaltenen Phospholipide (PL) enthalten sind, beträgt.

2. Das Verfahren nach Anspruch 1, wobei der zu *Mortierella sp.* gehörende Mikroorganismus ein Mikroorganismus der Untergattung *Mortierella* ist.

3. Das Verfahren nach Anspruch 2, wobei der zu *Mortierella sp.* gehörende Mikroorganismus *Mortierella alpina* ist.

4. Das Verfahren nach einem der Ansprüche 1 bis 3, wobei das Kultivieren weiter Kultivieren unter Bedingungen umfasst, die einen anfänglichen pH von 4 bis 6 beinhalten.

5. Das Verfahren nach einem der Ansprüche 1 bis 4, wobei das Kultivieren weiter Kultivieren in einem Medium umfasst, das 3,7 mM bis 147 mM eines Phosphatsalzes enthält.

6. Das Verfahren nach einem der Ansprüche 1 bis 5, wobei das Kultivieren weiter Kultivieren unter Rühren bei einer erforderlichen maximalen Rührkraft (KLA = (P/V)^{0,95}·VS^{0,67}) von 8,3 oder mehr umfasst.

7. Das Verfahren nach einem der Ansprüche 1 bis 6, wobei das Kultivieren weiter Kultivieren bei 10°C bis 25°C für 4 bis 18 Stunden nach der Anzucht der Zellen umfasst.

8. Das Verfahren nach einem der Ansprüche 1 bis 6, wobei das Kultivieren weiter Kultivieren bei 10°C bis 25°C für 4 bis 18 Stunden nach Kultivieren bei 28°C für 2 bis 20 Tage umfasst.

9. Das Verfahren nach einem der Ansprüche 1 bis 8, wobei das kultivierte Produkt eine Kulturlösung oder eine sterilisierte Kulturlösung ist oder daraus gesammelte kultivierte Zellen oder getrocknete Zellen davon umfasst.

10. Das Verfahren nach einem der Ansprüche 1 bis 9, wobei LCPUFA-PL mindestens ein Glycerophospholipid, ausgewählt aus der Gruppe bestehend aus Phosphatidylcholin, Phosphatidylserin, Phosphatidylethanolamin, Phosphatidylinositol, Phosphatidsäure und Cardiolipin, umfasst.

11. Das Verfahren nach einem der Ansprüche 1 bis 10, welches zur Herstellung von LCPUFA-PL verwendet wird, das als einen Bestandteil eine langkettige mehrfach ungesättigte Fettsäure (LCPUFA), ausgewählt aus der Gruppe bestehend aus mehrfach ungesättigten Omega-6-Fettsäuren, mehrfach ungesättigten Omega-3-Fettsäuren und mehrfach ungesättigten Omega-9-Fettsäuren, enthält.

12. Das Verfahren nach Anspruch 11, wobei die mehrfach ungesättigten Omega-6-Fettsäuren eine oder mehrere Fettsäuren, ausgewählt aus der Gruppe bestehend aus 11,14-Eicosadiensäure (20:2, ω6), 8,11,14-Eicosatriensäure (Dihomo-γ-linolensäure; 20:3, ω6), 5,8,11,14-Eicosatetraensäure (Arachidonsäure; 20:4, ω6), 13,16-Docosadiensäure (22:2, ω6), 7,10,13,16-Docosatetraensäure (22:4, ω6) und 4,7,10,13,16-Docosapentaensäure (22:5, ω6), sind.

13. Das Verfahren nach Anspruch 11, wobei die mehrfach ungesättigten Omega-3-Fettsäuren eine oder mehrere Fettsäuren, ausgewählt aus der Gruppe bestehend aus 11,14,17-Eicosatriensäure (α-Linolensäure; 20:3, ω3), 8,11,14,17-Eicosatetraensäure (20:4, ω3), 5,8,11,14,17-Eicosapentaensäure (20:5, ω3), 7,10,13,16,19-Docosapentaensäure (22:5, ω3) und 4,7,10,13,16,19-Docosahexaensäure (22:6, ω3), sind.

14. Das Verfahren nach Anspruch 11, wobei die mehrfach ungesättigten Omega-9-Fettsäuren eine oder mehrere Fettsäuren, ausgewählt aus der Gruppe bestehend aus 5,8,11-Eicosatriensäure (Mead'sche Säure; 20:3, ω9), 7,10,13-Docosatriensäure (22:3, ω9) und 4,7,10,13-Docosatetraensäure (22:4, ω9), sind.

15. Das Verfahren nach einem der Ansprüche 1 bis 10, welches zur Herstellung von LCPUFA-PL verwendet wird, das als einen Bestandteil LCPUFA, ausgewählt aus der Gruppe bestehend aus Arachidonsäure (5,8,11,14-Eicosatetraensäure) und Docosahexaensäure (4,7,10,13,16,19-Docosahexaensäure), enthält.

16. Das Verfahren nach einem der Ansprüche 1 bis 10, welches zur Herstellung von LCPUFA-PL verwendet wird, das als einen Bestandteil LCPUFA mit mindestens einer konjugierten Doppelbindung in ihrem Molekül enthält.

17. Das Verfahren nach einem der Ansprüche 1 bis 10, wobei das Zusammensetzungsverhältnis von Isopalmitinsäure, einer verzweigten Fettsäure, 5% oder weniger, bezogen auf die Gesamtmenge an Fettsäuren, die als Bestandteile aller in dem kultivierten Produkt enthaltenen Phospholipide (PL) enthalten sind, beträgt, und das Zusammensetzungsverhältnis von Arachidonsäure 13% oder mehr, bezogen auf die Gesamtmenge der Fettsäuren, beträgt.

18. Das Verfahren nach einem der Ansprüche 1 bis 10, wobei das Zusammensetzungsverhältnis von Isopalmitinsäure, einer verzweigten Fettsäure, 5% oder weniger, bezogen auf die Gesamtmenge an Fettsäuren, die als Bestandteile aller in dem kultivierten Produkt enthaltenen Phospholipide (PL) enthalten sind, beträgt und das Zusammensetzungsverhältnis von Isopalmitinsäure außerdem 1/6 oder weniger des Zusammensetzungsverhältnisses von Arachidonsäure, bezogen auf die Gesamtmenge der Fettsäuren, beträgt.

19. Das Verfahren nach einem der Ansprüche 1 bis 10, wobei LCPUFA-PL ein Arachidonsäure enthaltendes Phospholipid umfasst und die Menge an Arachidonsäure in allen in dem kultivierten Produkt enthaltenen Phospholipiden 11,8 mg oder mehr pro g getrockneter Zellen beträgt.

20. Das Verfahren nach einem der Ansprüche 1 bis 10, wobei LCPUFA-PL ein Dihomo-γlinolensäure (DGLA) enthaltendes Phospholipid umfasst und die Menge an DGLA in allen in dem kultivierten Produkt enthaltenen Phospholipiden 1,5 mg oder mehr pro g getrockneter Zellen beträgt.

21. Das Verfahren nach einem der Ansprüche 1 bis 9, wobei LCPUFA-PL ein Arachidonsäure enthaltendes Phosphatidylcholin umfasst und die Menge an Arachidonsäure, die als ein Bestandteil aller in dem kultivierten Produkt enthaltenen Phosphatidylcholine enthalten ist, 1,0 mg oder mehr pro g getrockneter Zellen beträgt.

22. Das Verfahren nach einem der Ansprüche 1 bis 9, wobei LCPUFA-PL ein Arachidonsäure enthaltendes Phosphatidylethanolamin umfasst und die Menge an Arachidonsäure, die als ein Bestandteil aller in dem kultivierten Produkt enthaltenen Phosphatidylethanolamine enthalten ist, 0,5 mg oder mehr pro g getrockneter Zellen beträgt.

23. Das Verfahren nach einem der Ansprüche 1 bis 9, wobei LCPUFA-PL ein Arachidonsäure enthaltendes Phosphatidylserin umfasst und die Menge an Arachidonsäure, die als ein Bestandteil aller in dem kultivierten Produkt enthaltenen Phosphatidylserine enthalten ist, 0,05 mg oder mehr pro g getrockneter Zellen beträgt.

24. Das Verfahren nach einem der Ansprüche 1 bis 10, wobei das kultivierte Produkt ein Arachidonsäure enthaltendes Phospholipid als einen Bestandteil und ein Arachidonsäure enthaltendes Triglycerid als einen Bestandteil enthält, wobei die Menge an als ein Bestandteil des Phospholipids vorliegender Arachidonsäure in einem Verhältnis von 0,2 oder mehr bezogen auf die Menge an als ein Bestandteil des Triglycerids vorliegender Arachidonsäure vorliegt.

## Revendications

1. Procédé pour produire un phospholipide contenant un acide gras polyinsaturé à chaîne longue en C₂₀ ou plus en tant que constituant (LCPUFA-PL), qui comprend :
(i) la culture d'un micro-organisme appartenant à *Mortierella sp.* dans lequel la culture est contrôlée afin de maintenir un pH entre 4 et 6 permettant ainsi l'accumulation intracellulaire de LCPUFA-PL dans le micro-organisme ; et
(ii) l'obtention de LCPUFA-PL à partir du produit cultivé du micro-organisme, dans lequel le rapport de composition d'acide isopalmitique, un acide gras ramifié, est de 5 % ou moins sur la base de la quantité totale d'acides gras présents en tant que constituants de l'ensemble des phospholipides (PL) contenus dans le produit cultivé.

2. Procédé selon la revendication 1, dans lequel le micro-organisme appartenant à *Mortierella sp.* est un micro-organisme du sous-genre *Mortierella.*

3. Procédé selon la revendication 2, dans lequel le micro-organisme appartenant à *Mortierella sp.* est *Mortierella alpina.*

4. Procédé selon l'une quelconque des revendications 1 à 3, dans lequel la culture comprend en outre la culture dans des conditions comprenant un pH initial de 4 à 6.

5. Procédé selon l'une quelconque des revendications 1 à 4, dans lequel la culture comprend en outre la culture dans un milieu contenant 3,7 mM à 147 mM d'un sel de phosphate.

6. Procédé selon l'une quelconque des revendications 1 à 5, dans lequel la culture comprend en outre la culture sous agitation à une puissance d'agitation maximale requise (KLA = (P/V)^{0,95}·VS^{0,67}) de 8,3 ou plus.

7. Procédé selon l'une quelconque des revendications 1 à 6, dans lequel la culture comprend en outre la culture à 10 °C à 25 °C pendant 4 à 18 heures après culture des cellules.

8. Procédé selon l'une quelconque des revendications 1 à 6, dans lequel la culture comprend en outre la culture à 10 °C à 25 °C pendant 4 à 18 heures après culture à 28 °C pendant 2 à 20 jours.

9. Procédé selon l'une quelconque des revendications 1 à 8, dans lequel le produit cultivé est une solution de culture ou une solution de culture stérilisée, ou comprend des cellules cultivées collectées à partir de celle-ci ou des cellules séchées de celle-ci.

10. Procédé selon l'une quelconque des revendications 1 à 9, dans lequel LCPUFA-PL comprend au moins un glycérophospholipide choisi dans le groupe constitué de la phosphatidylcholine, la phosphatidylsérine, la phosphatidyléthanolamine, le phosphatidylinositol, l'acide phosphatidique et la cardiolipine.

11. Procédé selon l'une quelconque des revendications 1 à 10, qui est utilisé pour la production de LCPUFA-PL contenant, en tant que constituant, un acide gras polyinsaturé à chaîne longue (LCPUFA) choisi dans le groupe constitué d'acides gras polyinsaturés oméga-6, d'acides gras polyinsaturés oméga-3 et d'acides gras polyinsaturés oméga-9.

12. Procédé selon la revendication 11, dans lequel les acides gras polyinsaturés oméga-6 sont un ou plusieurs acides gras choisis dans le groupe constitué de l'acide 11,14-eicosadiénoïque (20:2, ω6), l'acide 8,11,14-eicosatriénoïque (acide dihomo-γ-linolénique ; 20:3, ω6), l'acide 5,8,11,14-eicosatétraénoïque (acide arachidonique ; 20:4, ω6), l'acide 13,16-docosadiénoïque (22:2, ω6), l'acide 7,10,13,16-docosatétraénoïque (22:4, ω6) et l'acide 4,7,10,13,16-docosapentaénoïque (22:5, ω6).

13. Procédé selon la revendication 11, dans lequel les acides gras polyinsaturés oméga-3 sont un ou plusieurs acides gras choisis dans le groupe constitué de l'acide 11,14,17-eicosatriénoïque (acide α-linolénique ; 20:3, ω3), l'acide 8,11,14,17-eicosatétraénoïque (20:4, ω3), l'acide 5,8,11,14,17-eicosapentaénoïque (20:5, ω3), l'acide 7,10,13,16,19-docosapentaénoïque (22:5, ω3) et l'acide 4,7,10,13,16,19-docosahexaénoïque (22:6, ω3).

14. Procédé selon la revendication 11, dans lequel les acides gras polyinsaturés oméga-9 sont un ou plusieurs acides gras choisis dans le groupe constitué de l'acide 5,8,11-eicosatriénoïque (acide de Mead ; 20:3, ω9), l'acide 7,10,13-docosatriénoïque (22:3, ω9) et l'acide 4,7,10,13-docosatétraënoïque (22:4, ω9).

15. Procédé selon l'une quelconque des revendications 1 à 10, qui est utilisé pour la production de LCPUFA-PL contenant, en tant que constituant, un LCPUFA choisi dans le groupe constitué de l'acide arachidonique (acide 5,8,11,14-eicosatétraénoïque) et l'acide docosahexaénoïque (4,7,10,13,16,19-docosahexaénoïque).

16. Procédé selon l'une quelconque des revendications 1 à 10, qui est utilisé pour la production de LCPUFA-PL contenant, en tant que constituant, un LCPUFA ayant au moins une double liaison conjuguée dans sa molécule.

17. Procédé selon l'une quelconque des revendications 1 à 10, dans lequel le rapport de composition d'acide isopalmitique, un acide gras ramifié, est de 5 % ou moins sur la base de la quantité totale d'acides gras présents en tant que constituants de l'ensemble des phospholipides (PL) contenus dans le produit cultivé, et le rapport en composition d'acide arachidonique est de 13 % ou plus sur la base de la quantité totale d'acides gras.

18. Procédé selon l'une quelconque des revendications 1 à 10, dans lequel le rapport en composition d'acide isopalmitique, un acide gras ramifié, est de 5 % ou moins sur la base de la quantité totale d'acides gras présents en tant que constituants de l'ensemble des phospholipides (PL) contenus dans le produit cultivé, et le rapport en composition d'acide isopalmitique est également 1/6 ou moins du rapport en composition d'acide arachidonique sur la base de la quantité totale d'acides gras.

19. Procédé selon l'une quelconque des revendications 1 à 10, dans lequel LCPUFA-PL comprend un phospholipide contenant de l'acide arachidonique, et la quantité d'acide arachidonique dans l'ensemble des phospholipides contenus dans le produit cultivé est de 11,8 mg ou plus par g de cellules séchées.

20. Procédé selon l'une quelconque des revendications 1 à 10, dans lequel LCPUFA-PL comprend un phospholipide contenant de l'acide dihomo-γ-linolénique (DGLA), et la quantité de DGLA dans l'ensemble des phospholipides contenus dans le produit cultivé est de 1,5 mg ou plus par g de cellules séchées.

21. Procédé selon l'une quelconque des revendications 1 à 9, dans lequel LCPUFA-PL comprend une phosphatidylcholine contenant de l'acide arachidonique, et la quantité d'acide arachidonique présent en tant que constituant de l'ensemble des phosphatidylcholines contenues dans le produit cultivé est de 1,0 mg ou plus par g de cellules séchées.

22. Procédé selon l'une quelconque des revendications 1 à 9, dans lequel LCPUFA-PL comprend une phosphatidyléthanolamine contenant de l'acide arachidonique, et la quantité d'acide arachidonique présent en tant que constituant de l'ensemble des phosphatidyléthanolamines contenues dans le produit cultivé est de 0,5 mg ou plus par g de cellules séchées.

23. Procédé selon l'une quelconque des revendications 1 à 9, dans lequel LCPUFA-PL comprend une phosphatidylsérine contenant de l'acide arachidonique, et la quantité d'acide arachidonique présent en tant que constituant de l'ensemble des phosphatidylsérines contenues dans le produit cultivé est de 0,05 mg ou plus par g de cellules séchées.

24. Procédé selon l'une quelconque des revendications 1 à 10, dans lequel le produit cultivé contient un phospholipide contenant de l'acide arachidonique en tant que constituant et un triglycéride contenant de l'acide arachidonique en tant que constituant, dans lequel la quantité d'acide arachidonique présent en tant que constituant du phospholipide est dans un rapport de 0,2 ou plus par rapport à la quantité d'acide arachidonique présent en tant que constituant du triglycéride.
